# EUROPEAN PATENT APPLICATION

(11) **EP 0 858 994 A1**
(43) Date of publication of application: **19.08.1998**
(21) Application number: 98200383.2
(22) Date of filing: 06.02.1998
(51) Int. Cl.: C07C 307/06, H01M 6/16, H01M 10/40

(54) **New ionically conductive material with improved conductivity and stability**

(30) Priority: 07.02.1997 CA 2197056
(71) Applicant: HYDRO-QUEBEC, Montréal Québec H2Z 1A4 (CA); Universite de Montreal, Montréal, Québec H3T 1C5 (CA)
(72) Inventor: Choquette, Yves, Sainte-Julie, Quebec J3E 1P4 (CA); Armand, Michel, Montreal, Quebec H3T 1N2 (CA); Gravel, Denis, St-Lambert, Quebec J4S 1L3 (CA); Slougui, Nasreddine, Montreal, Quebec H2B 1Z7 (CA); Brisard, Gessie, Sherbrooke, Quebec J1H 2S2 (CA); Parent, Michel, Liniere, Beauce, Quebec G0M 1J0 (CA); Brouillette, Dany, Montreal, Quebec H3K 1R7 (CA); Perron, Gérald, Boucherville, Quebec J4B 6G1 (CA); Labreche, Christian, Montreal (Quebec)H2M 2H8 (CA); Prud'Homme, Jacques, Lavel, Quebec H7V 3P1 (CA)
(74) Representative: Guerre, Dominique

(57) **Abstract**

Sulfamides or mixtures thereof having the formula

R₁ R₂ NSO₂ N R₃ R₄

wherein one to three R substituents are methyl groups, the remainder being ethyl groups, or one R is a methoxyethyl, the remaining R being methyl or ethyl groups. They are useful as electrolytic composition with outstanding conductivity and aprotic solvents. An electrolyte comprising them is highly useful in a lithium battery, a supercapacity type device, an electrochomic device or as a component of a solvent.

## Description

### BACKGROUND OF INVENTION

### (a) Field of the Invention

This invention relates to an ionically conductive material with improved conductivity and stability. More particularly, the present invention is concerned with sulfamide derivatives of the formula:

R₁ R₂ NSO₂ N R₃ R₄

wherein R₁, R₂, R₃ and R₄ are methyl, the remaining groups being ethyl groups, or one of R₁, R₂, R₃ and R₄ is a methoxyethyl, the remaining groups being methyl or ethyl groups, which are used as aprotic solvents which can be used as media for organic reactions and/or synthesis; as ionically conductive material when a salt is added as a solute to a sulfamide of the above formula; as solid or gel polymer electrolytes useful for electrochemical applications, such as lithium type batteries or so-called rocking chair or lithium ion batteries, the electrolytes being obtained by adding a sulfamide of the above formula with a salt, to a solid or liquid polymer.

### (b) Description of the Prior Art:

Sulfamides (R₁R₂NSO₂NR₃R₄) wherein R₁, R₂, R₃ and R₄ represent alkyl groups and their derivatives are used in many chemical and biological applications. Richey et al (*Journal of Organic Chemistry*, Vol. 52, No 4, (1987) p. 479-483) report that when used as solvents they are particularly stable toward polar organometallic compounds as well as toward other strongly basic and nucleophilic reagents. It has been found by Richey et al that organomagnesium compounds are indefinitely stable in tetraethylsulfamide identified as TES when R₁, R₂, R₃ and R₄ are all ethyl groups and that the half-life of butyllithium in TES exceeds 15 minutes while in other solvent the half-lives are less than 5 minutes.

Sulfamides are also used in the field of pesticides (U.S. Patent No. 5,596,017) and in biological applications. A method for preparing sulfamide compounds is described in U.S. Patent No. 5,506,355. U.S. Patent No. 3,879,528, U.S. Patent No. 5,539,102, and U.S. Patent No. 3,923,886 describe routes and processes for producing sulfamide compounds for those applications.

Sulfamides are also well known for thee capability to increase the flame resistance of polymers. U.S. Patent Nos. 3,888,819 and 3,915,931 describe sulfamide compositions and a process for that particular application.

Proton-vacancy conducting polymers based on polyethylene oxide (PEO) and sulfamide-type salts were also reported in the literature by Bermudez and al. (Electrochimica Acta, Vol. 37, No 9, (1992) pp 1603-1609) as a novel basic proton conducting polymer. The presence of the sulfamide-type salt is a key issue of this concept for proton conducting polymer.

In the field of lithium battery, battery performances of PEO polymer modified with sulfonamide end groups provide better conductivity than the PEO system alone. It was reported for example by Ito et al (Solid State Ionics, 86-88, (1996), p 325-328) that PEO having alkyl-sulfonamide end groups such as PEO₁₀₀₀-(NKSO₂Me)₂ result in a higher ionic conductivity as compared to PEO₁₀₀₀. The authors explain this higher conductivity by the higher dissociation constant of the terminal sulfonamide.

The identification of new solvents having high dielectric constants, and solvatation powers (adequate high donor (DN) and acceptor (AN) numbers) as well as low viscosities is a paramount for high energy lithium cell development operating at ambient temperature. Mixture of solvents intended to improve overall battery performances may also be of utility as exemplified for instance in U.S. Patent No 4,129,691. These solvents could be used in liquid lithium batteries as well as in gel electrolyte containing polymers networks (cf. U.S. Patent Nos. 4,792,504 and 5,501,920).

U.S. Patent Nos. 4,851,307 and 5,063,124 describe new ionically conductive materials which comprise a salt dissolved in a solvent or mixed therewith in variable amounts of this solvent with a polymer having solvating groups; this solvent being a sulfamide derivative of general formula:

R₁ R₂ NSO₂ N R₃ R₄

wherein R₁, R₂, R₃ and R₄ are each independently a C(1-10) alkyl group or a C(1-10) oxaalkyl group. A representative example of this group is R₁ = R₂, = R₃ = R₄ = ethyl, abbreviated TES, will be considered the reference molecule in the description which follows.

Neither U.S. Patent No. 4,851,307 or U.S. Patent No. 5,063,124 report or claim specific chemical or electrochemical properties or benefits by selecting specific R groups nor describe their relative position on the two nitrogen atoms.

### SUMMARY OF INVENTION

It is an object of the present invention to provide a new family of sulfamides having different Rᵢ groups and which possess different chemical properties, making them useful for different applications such as in the field of lithium batteries and other organic chemistry in general.

It is another object of the present invention to provide a family of sulfamide derivatives that can be used as aprotic solvents for organic reactions and/or synthesis.

It is another object of the present invention to provide derivatives of the present invention to provide sulfamide derivatives which can be used to produce ionically conductive materials, solids or gel polymer electrolytes or solid and liquid electrolytes.

It is another object of the present invention to provide sulfamide compositions whose chemical and electrochemical properties are especially advantageous for applications as solvent for chemical and electrochemical reactions.

It is another object of the present invention to provide sulfamides whose properties are especially advantageous for applications as solvent for chemical and electrochemical reactions, and especially for liquid and/or solid and/or gel lithium batteries or rocking chair batteries.

The present invention relates to sulfamides or mixtures for use in an electrolytic composition or as aprotic solvents, said sulfamides having the general formula:

R₁R₂NSO₂NR₃R₄

wherein one to three R substituents are methyl groups, the remainder being ethyl groups, or one R is a methoxyethyl, the remaining R being methyl or ethyl groups.

When R₁ = R₂ = R₃ = methyl, R₄ = ethyl, the sulfamide has a dielectric constant of 57, and a melting point of -33°C.

When R₁ = R₂ = methyl, R₃ = R₄ = ethyl, the sulfamide has a dielectric constant of 40.5, a melting point of -24°C and a glass transition temperature of -122°C.

When R₁ = R₃ = methyl, R₂ = R₄ = ethyl, the sulfamide has a dielectric constant of 44, a melting point of -56°C and a glass transition temperature of -130°C.

When R₁ = R₂ = R₃ methyl, R₄ = methoxy-ethyl, the sulfamide has a dielectric constant of 41.7 and a glass transition temperature of -110°C.

When R₁ = R₂ = R₃ = ethyl, R₄ = methoxy-ethyl, the sulfamide has a dielectric constant of 29, a glass transition temperature of -118°C and a stability window extending from 0 to 5 V. vs. lithium.

The invention also relates to an electrolytic composition which comprises at least one sulfamide composition defined above and at least one dissociable salt in solution therein. The sulfamide may be in admixture with a co-solvent, and the salt may be a lithium salt.

According to a preferred embodiment the lithium salt is selected from the group consisting of LiBF₄, LiPF₆, Li(R_{F}SO₂)2N Li(R_{F}SO₂)₃C where R_{F} is fluorine or a fluorinated carbon radical having 1 - 4 carbon atoms.

The co-solvent may be chosen from the group consisting of dimethyl ethers of ethylene glycol, diethylene glycol, triethylene glycol, and/or ethylene carbonate, propylene carbonate, dimethyl carbonate, diethyl carbonate γ-butyrolactone.

In the electrolytic composition the sulfamide may be in admixture with an aprotic polymer, such as polyethers comprising at least 70% ethylene oxide units, poly(methyl methacrylate), poly(acrylonitrile), and copolymers containing at least 70% vinylidene fluoride units.

The sulfamide to polymer ratio is preferably less than or equal to 20% and the sulfamide acts as a plasticizer.

The solvent to polymer ratio may be greater than or equal to 50% and the resulting mixture then constitutes a gel.

The invention also relates to a lithium battery wherein the electrolyte, alone or as a component of composite electrodes comprises an electrolytic composition as defined above. In this battery the negative electrode may contain either metallic lithium, a lithium alloy, a carbon intercalation compound or a low voltage intercalation oxide or nitride, and may contain a high voltage intercalation electrode derived from vanadium, manganese, cobalt or nickel oxides, iron or manganese iron phosphate or pyrophosphate. The positive electrode may also contain a polydisulfide or sulfur mixed with a conductive additive such as high surface area carbon.

The invention also relates to a supercapacity type device wherein the electrolyte comprises a composition as defined above, wherein the electrodes may contain high surface area carbon.

The invention also relates to an electrochomic device wherein the electrolyte comprises a composition as defined above, and wherein one of the electrodes may be a thin film of tungsten trioxide.

The invention also relates to a solvent composition for use as media containing a sulfamide as defined above alone or in admixtures with organic solvents. Such composition may include co-solvents such as aromatic hydrocarbons, or halocarbons, and may be used as media for organic reactions comprising carbanion preparation, Friedel & Craft reactions, Diels & Alder or Michael reactions, cationic or anionic polymerizations.

It is known to the specialist in the field that:
- shortening of a alkyl groups usually results in molecules with less conformational freedom thus increased melting points, viscosities or glass transition temperatures Tg for glass-forming compositions. Examples are for instance the well know propylene carbonate, tuning into a glass below -40°C and ethylene carbonate (melting point Tₘₚ= 39°C); dimethylcarbonate, Tₘₚ= 4°C, diethylcarbonate, Tₘₚ= -43°C; acetonitrile Tₘₚ= -48°C, propionitrile Tₘₚ= -93°C. Similarly, tetramethylsulfamide abbreviated TMS melts at 73°C;
- shorter alkyl groups, especially methyl have reduced electron-releasing properties, and the donor numbers (DN) in Gutmann's definition [V. Gutmann, Electochimica Acta 21, 661-670, (1976)] of the corresponding molecules built from these alkyl chains are lower (i.e. acetonitrile, DN = 14.1, butyronitrile DN = 16.6). Conversely, the acceptor number (AN) is usually moderately affected by chain length (ethanol: AN = 37, *n*-butanol AN = 36.8);
   - the oxidative stability of ether groups does not extend beyond 3.8 Volts vs. the lithium reference electrode;
   - an increase in dielectric constant is usually accompanied by in increase in reactivity, as the dipoles bear a higher charge and/or are closer to the outer part of the molecule, thus are more exposed to reactants.

Properties varying progressively could be expected when going from TES to TMS with sequential replacement of ethyl by methyl groups. Surprisingly, outstanding behavior was observed on these compounds and those containing the ether linkage in methoxyethyl substituents. The properties were not foreseen or anticipated by one skilled in the art, thus clearly establishing the novelty aspect of the present invention.

The principal properties of interest as solvent for chemical and electrochemical applications are: the liquid temperature range, the dielectric constant (e), the donor (DN) and acceptor (AN) numbers, the glass transition temperature, (Tg) alone or in admixtures, especially with polar polymers. For electrochemical use, the property values of the first three parameters allow for an increase in the specific conductivity. The electrochemical stability window towards metallic lithium and the potential beyond which solvent oxidation takes place is also a very important property for battery application and must be as wide as possible.

A good solvent for liquid lithium batteries must have a low viscosity, a high boiling point, a low melting point and should be able to dissolve a large quantity of salt (high DN and AN). For lithium solid polymer batteries, the solvent can be used as a plasticizer (small amount of solvent is added to the polymer, typically less than 50% volume). In this particular application a good plasticizer will lower the Tg of the polymer matrix and its crystallinity. For gel electrolytes (large volume fraction, 50% or higher, of the solvent immobilized by a compatible polymer matrix), all the mentioned properties are important since ion transport mechanism (specific conductivity) can be dependent on both media (solvent and polymer).

### DRAWINGS

The invention is illustrated but is not limited by the following drawings in which:
FIGURE 1 is a graph showing the increase in conductivity of NES-I-4 as compared to TES;
FIGURE 2 illustrates the conductivity of PEO-LiTFSI mixture, POE-LiTFST-NES-I-6 (9% weight) mixture and PEO-LiTFSI-TES (8% weight) mixture, all systems having a 35% weight LiTFSI;
FIGURE 3 is a graph comparing the electrochemical stability of NES-II-17 and that of TES; and
FIGURE 4 is a graph comparing the capacities of cells having NES-I-3 and NES-II-65 with cells having TES under cycling.

The invention will also be illustrated by means of the following non limiting examples.

### EXAMPLES

### Example 1

The following table summarizes some of the properties of the new sulfamides (Me = methyl, Et = ethyl, s is the conductivity in mS/cm, e the dielectric constant, DN the donor number and AN the acceptor number according to Gutmann's definition; *n.m*.: not measurable; *n*. *misc*.: not miscible) and show the outstanding behavior of these compounds:

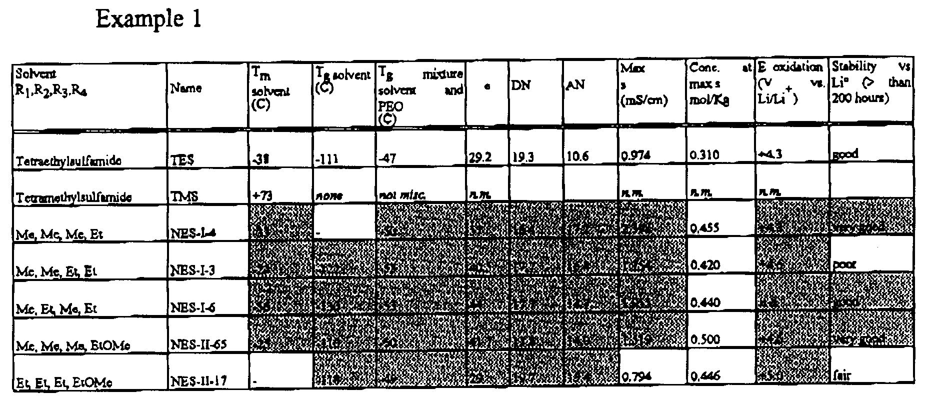

### Example 2

In this example, the conductivity of a solution containing 0.33 mol/kg of LiTFSI in the solvent NES-I-4 (which have the highest dielectric constant) is compared to the conductivity of the TES-LiTFSI (TESA) system at the same concentration of salt. Conductivity data are measured at different temperatures (from -20°C to 55°C) with a built in-house bridge similar to the Beckman model RC-15 commercial bridge. Platinized platinum Orion (Boston, USA) cells are used and resistance measurements are made at 1 and 3 kHz and extrapolated to 0 kHz.

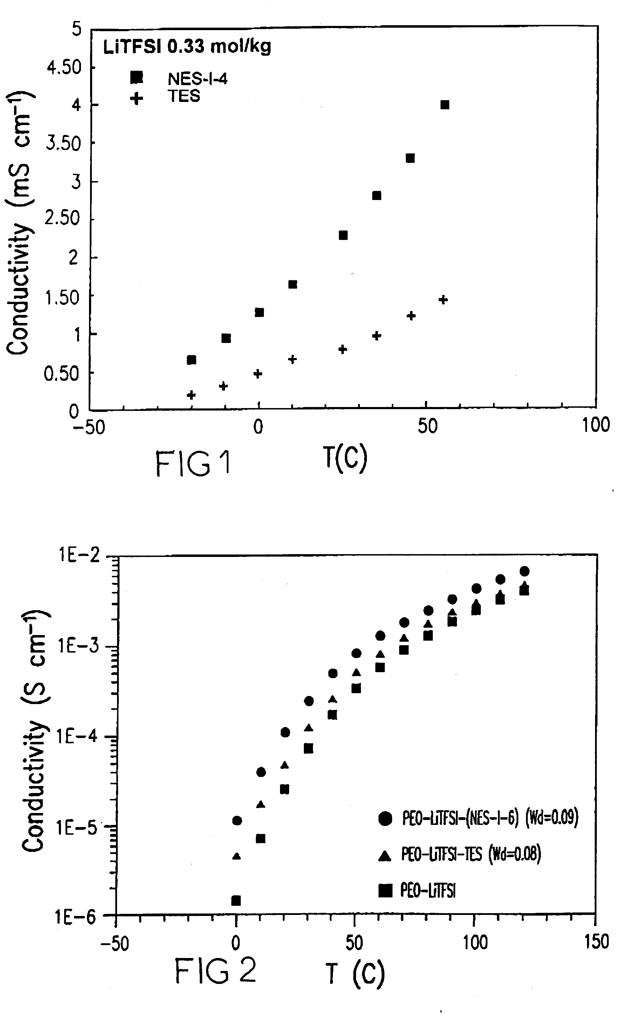

From FIGURE 1 it is noted that replacement of 3 ethyl groups of TES by 3 methyl groups (NES-I-4) considerably increases the conductivity, more than three times for all temperatures.

### Example 3

In this example, the conductivity of PEO-LiTFSI mixtures are illustrated. Samples are prepared as follows: 1) PEO (Mw = 4 x 10⁶) and LiTFSI are dissolved in acetonitrile; 2) the solution obtained is coated on an inert support and then dried under partial vacuum (40 mm Hg) for 2-3 hours at ambient temperature and 3) the electrolyte film is dried under vacuum at 140°C for 48 hours. Samples having plasticizer or diluent NES-I-6 or TES are prepared similarly. NES-I-6 or TES are added to the mixture of PEO-LiTFSI in acetonitrile and the same procedure as above is respected except that the final drying step is done under 2 mm Hg during 48h at ambient temperature. NES-I-6 or TES concentration in the final solid electrolyte film is controlled by RMN ¹H.

The SPE films are then disposed between two stainless steel electrodes. Conductivity cells are introduced in an air-tight cell holder that permit electrical contact. The conductivity measurements are performed in an Instron (Burlington, Ont., Canada) temperature chamber equipped with a computer-driven controller. Impedance data were collected at intervals of 10°C over the frequency range 5Hz-13MHz using a mode 4192A Hewlett-Packard impedance analyser.

The conductivity of the PEO-LiTFSI-(NES-I-6) mixture is around 2 times higher as compared to PEO-LiTFSI-TES and close to an order of magnitude (at 0°C) higher compared to the PEO-LiTFSI system.

### Example 4

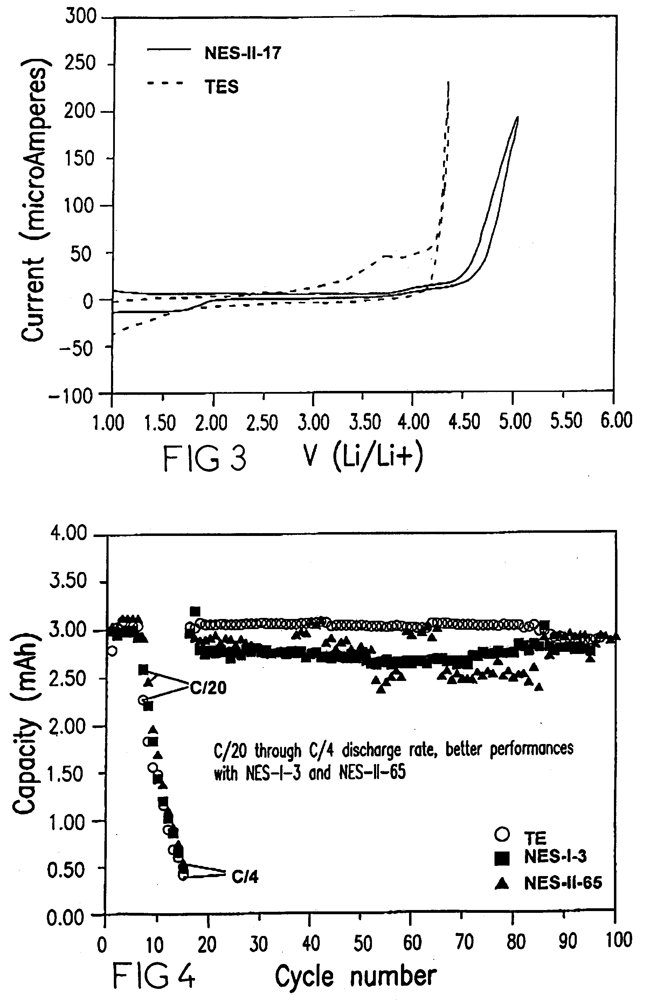

In this example, the electrochemical stability of NES-II-17 is compared to TES for the same concentration of LiTFSI introduced in the solution (0.37 M). A conventional three-electrode cell is used for that study and the working electrode is a glassy carbon rod embedded in Kel-F. Current responses in function of voltage scanning (scan rate = 20 mV/sec) are recorded and the results are presented in Figure 3. Massive solvent oxidation occurs near 5 V for NES-II-17 which is almost 700 mV more noble than for TES. NES-II-17 is therefore a better candidate for high voltage-energy lithium batteries.

### Example 5

In this example, the effect of different sulfamides on the power and cyclability of lithium polymer cells is compared. The three cells are made of a lithium anode (35 mm thick) supported on a thin sheet of nickel and a composite cathode whose composition by volume is about 40% TiS₂, 10% acetylene black and 50% of an ethylene oxide copolymer. This copolymer includes about 80% ethylene oxide as described in the following patents: EPO 0,013,199; U.S. 4,578,326; and U.S. 4,758,483, to which there is added the lithium salt LiTFSI in a ratio of 30/1. The cathode of effective capacity near 3C/cm² is placed on a thin nickel collector. The thickness of the separator of the polymer electrolyte is 15 mm and the latter is also made of an ethylene oxide base polymer. The batteries with useful surface of 3.89 cm² are assembled by hot pressing at 80°C. The sulfamide is added to the separator and the cathode just before the hot pressing step and the molecular sulfamide ratio introduced is 1:1 compared to the amount of LiTFSI in the cell.

The batteries are cycled at 25°C at constant current (both charge and discharge) at around C/40 rate and the power performance in discharge of these cells is obtained between cycles 7 to 15. Figure 4 show that cells having NES-I-3 and NES-II-65 retain higher capacity for C/x rate where x = 20 through 4 compared to TES. The cyclability at C/40 for all the cells is very good and similar.

## Claims

1. Sulfamides or mixtures for use in an electrolytic composition or as aprotic solvents, said sulfamides having the general formula:
R₁R₂NSO₂NR₃R₄
wherein one to three R substituents are methyl groups, the remainder being ethyl groups, or one R is a methoxyethyl, the remaining R being methyl or ethyl groups.

2. Sulfamide according to claim 1, characterized in that R₁ = R₂ = R₃ = methyl, R₄ = ethyl, having a dielectric constant of 57, and a melting point of -33°C.

3. Sulfamide according to claim 1, characterized in that R₁ = R₂ = methyl, R₃ = R₄ = ethyl, having a dielectric constant of 40.5, a melting point of -24°C and a glass transition temperature of -122°C.

4. Sulfamide according to claim 1, characterized in that R₁ = R₃ = methyl, R₂ = R₄ = ethyl, having a dielectric constant of 44,. a melting point of -56°C and a glass transition temperature of -130°C.

5. Sulfamide according to claim 1, characterized in that R₁ = R₂ = R₃ methyl, R₄ = methoxy-ethyl, having a dielectric constant of 41.7 and a glass transition temperature of -110°C.

6. Sulfamide according to claim 1, characterized in that R₁ = R₂ = R₃ = ethyl, R₄ = methoxy-ethyl, having a dielectric constant of 29, a glass transition temperature of -118°C and a stability window extending from 0 to 5 V. vs. lithium.

7. Electrolytic composition which comprises at least one sulfamide composition according to claim 1 and at least one dissociable salt in solution therein.

8. Electrolytic composition according to claim 7 wherein said sulfamide is in admixture with a co-solvent.

9. Electrolytic composition according to claim 7 wherein the salt is a lithium salt.

10. Electrolytic composition according to claim 8 wherein the lithium salt is selected from the group consisting of LiBF₄, LiPF₆, Li(R_{F}SO₂)2N Li(R_{F}SO₂)₃C where R_{F} is fluorine or a fluorinated carbon radical having 1 - 4 carbon atoms.

11. Electrolytic composition according to claim 8 wherein the co-solvent is chosen from the group consisting of dimethyl ethers of ethylene glycol, diethylene glycol, triethylene glycol, and/or ethylene carbonate, propylene carbonate, dimethyl carbonate, diethyl carbonate g-butyrolactone.

12. Electrolytic composition according to claim 7 wherein said sulfamide is in admixture with an aprotic polymer.

13. Electrolytic composition according to claim 12 wherein the aprotic polymer is selected from polyethers comprising at least 70% ethylene oxide units, poly(methyl methacrylate), poly(acrylonitrile), and copolymers containing at least 70% vinylidene fluoride units.

14. Electrolytic composition according to claims 11 and 13 wherein the sulfamide to polymer ratio is less than or equal to 20% and the sulfamide acts as a plasticizer.

15. Electrolytic composition according to claims 11 and 13 wherein the solvent to polymer ratio is greater than or equal to 50% and the resulting mixture constitutes a gel.

16. Electrolytic composition according to claims 11 and 13 wherein the solvent to polymer ratio is less than 50% and the resulting mixture is a highly plasticized polymer base system.

17. Lithium battery wherein the electrolyte, alone or as a component of composite electrodes comprises an electrolytic composition according to claims 7 to 16.

18. Lithium battery according to claim 17 wherein the negative electrode contains either metallic lithium, a lithium alloy, a carbon intercalation compound or a low voltage intercalation oxide or nitride.

19. Lithium battery according to claims 17 and 18 wherein the positive electrode contains a high voltage intercalation electrode derived from vanadium, manganese, cobalt or nickel oxides, iron or manganese iron phosphate or pyrophosphate.

20. Lithium battery according to claims 17 and 18 wherein the positive electrode contains a polydisulfide or sulfur mixed with a conductive additive such as high surface area carbon.

21. Supercapacity type device wherein the electrolyte comprises a composition according to claims 7 to 16.

22. Surpercapacity type device according to claim 21 wherein the electrodes contain high surface area carbon.

23. Electrochomic device wherein the electrolyte comprises a composition according to claims 7 to 16.

24. Electrochomic device to claim 23 wherein one of the electrodes is a thin film of tungsten trioxide.

25. Solvent composition for use as media containing a sulfamide according to claims 1 to 5 alone or in admixtures with organic solvents.

26. Solvent composition according to claim 25 wherein the co-solvents are like aromatic hydrocarbons, or halocarbons.

27. Solvent composition according to claims 25 and 26 for use as media for organic reactions comprising carbanion preparation, Friedel & Craft reactions, Diels & Alder or Michael reactions, cationic or anionic polymerizations.
